# EUROPEAN PATENT SPECIFICATION

(11) **EP 3 181 165 B1**
(45) Date of publication and mention of the grant of the patent: **15.08.2018**
(21) Application number: 16204186.7
(22) Date of filing: 14.12.2016
(51) Int. Cl.: A61M 1/16, A61M 1/32, A61M 1/34, A61M 1/10, A61M 1/36

(54) **APPARATUS FOR THE DECAPNEIZATION OF BLOOD**
VORRICHTUNG ZUR DECAPNEISIERUNG VON BLUT
APPAREIL D'ÉLIMINATION DU GAZ CARBONIQUE DANS LE SANG

(30) Priority: 18.12.2015 IT UB20159389
(43) Date of publication of application: 21.06.2017
(73) Proprietor: Eurosets S.r.l., 41036 Medolla (MO) (IT)
(72) Inventor: Petralia, Antonio, 48022 Lugo (RA) (IT); Ghelli, Nicola, 40018 S. Pietro in Casale (BO) (IT); Fontanili, Paolo, 42015 Correggio (RE) (IT)
(74) Representative: Zoli, Filippo

(56) References cited:
- WO-A2-95/11709
- JP-A- 2007 167 672

## Description

The present invention relates to an apparatus for the decapneization of blood.

As known, decapneization is a substitutive respiratory therapy during which oxygen is supplied to the blood and, at the same time, carbon dioxide in excess is eliminated.

This type of therapy is generally implemented by means of the so-called "oxygenators", which are precisely suitable for removing CO2 from the incoming blood and enriching it with oxygen.

Therapies are also known for the filtering of blood providing for the removal of waste substances that have accumulated in the blood for various reasons such as: pathological causes, surgical causes, administration of substances or other.

These therapies are performed by means of so-called "blood-filtering machines", which implement the functions normally carried out by healthy kidneys in correct working conditions.

One therapy of this type is, e.g., CRRT (acronym for Continuous Renal Replacement Therapy).

This kind of machines, to which the patient remains connected also for long periods of time, comprise a filtering device, through which the blood to be treated is conveyed and which is connected to the patient by means of a supply line, adapted to transport the blood to be purified that is drawn from a vein of the patient, and a return line, by means of which the purified blood is reinfused into the patient.

The extra-renal therapy often needs to be associated with the substitutive respiratory therapy.

The relevant machines generally comprise a circuit that is composed of a supply line of the blood to be treated, of a device for the oxygenation of blood, of a filtering device, adapted to remove the waste substances present in the patient, and of a return line for the reinfusion of the treated blood into the patient. The blood is conveyed along the supply line by means of the pumping means of the type of a peristaltic pump.

Some types of apparatus for the decapneization of blood are known from EP 1 415 673 and EP 1 698 362.

EP 1 415 673 describes an apparatus for the decapneization in which the blood to be treated is pushed, by means of a pump, in sequence through the oxygenator and the hemofilter before being reinfused into the patient.

EP 1 698 362 describes, on the other hand, an apparatus for the decapneization in which the oxygenator and the hemofilter are locked together to form a single body piece.

These apparatuses of known type do have some drawbacks.

One drawback is the fact that they allow the use thereof only for limited periods of time.

Another drawback consists in the fact that the blood running through the supply line is constrained to pass through both the oxygenator and the hemofilter in sequence. This makes, however, impractical the simultaneous use of the two devices for a long time, because the oxygenator and the hemofilter are characterized by different operating ranges, in particular the oxygenator has a greater operating range (4/5 days approx.) than the hemofilter (24 hours approx.).

This drawback implies using the machine for the decapneization depending on the operating range of the hemofilter or replacing or by-passing the hemofilter while maintaining, at the same time, the oxygenator functioning.

In the cases in which the circuit configuration makes this possible, the hemofilter can be by-passed, which can, however, lead to the formation of blood stasis and clots which can impair the correct operation of the oxygenator. WO95/11709 discloses an extracorporeal blood oxygenation system using roller and centrifugal pumps. The main aim of the present invention is to provide an apparatus for the decapneization of blood which allows controlling the filtration of blood irrespective of the oxygenation thereof.

Within this aim, one object of the present invention is to maintain the oxygenator functioning also during the replacement or maintenance of the hemofilter.

More particularly, one object of the present invention is to avoid, during the operation of the oxygenator and the simultaneous exclusion of the hemofilter, the formation of blood stasis.

Another object of the present invention is to provide an apparatus for the decapneization of blood which allows to overcome the mentioned drawbacks of the prior art within the ambit of a simple, rational, easy, effective to use and affordable solution.

The above mentioned objects are achieved by the present apparatus according to claim 1.

Other characteristics and advantages of the present invention will become better evident from the description of a preferred, but not exclusive, embodiment of an apparatus for the decapneization of blood, illustrated by way of an indicative, but non-limiting, example in the accompanying drawings, wherein:

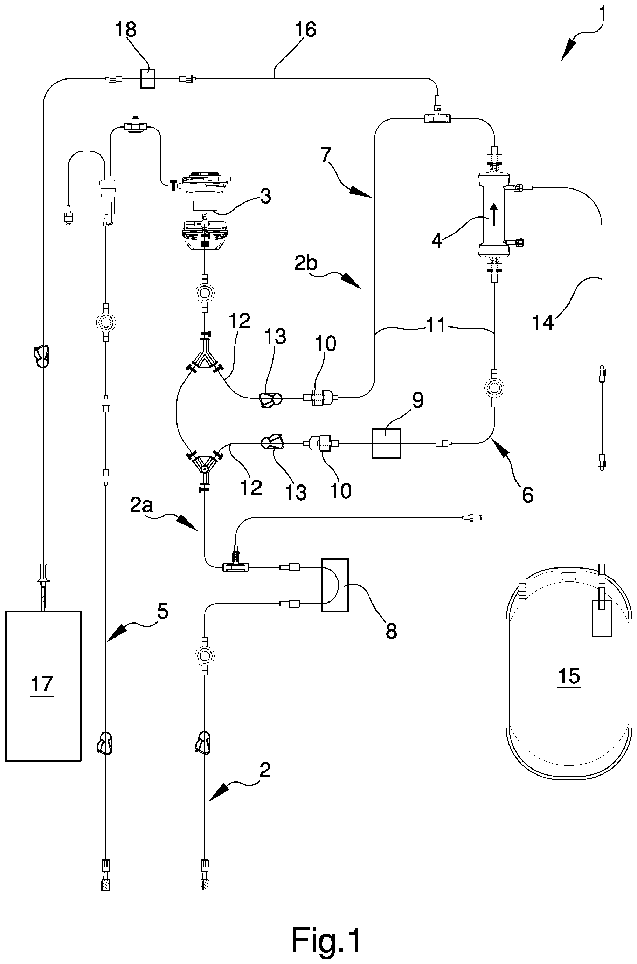

Figure 1 is a schematic view of an apparatus according to the invention.

With particular reference to such illustrations, globally indicated with the reference numeral 1 is an apparatus for the decapneization of blood.

The apparatus 1 comprises a supply line 2 of the blood to be treated taken from a patient, at least a return line 5 adapted to transport the treated blood to the patient, at least an oxygenation device 3 of the blood and at least a filtering device 4 positioned between the supply line 2 and the return line 5.

The apparatus 1 also comprises first pumping means 8 adapted to convey the blood to be treated along the supply line 2 towards at least one of the oxygenation device 3 and the filtering device 4.

According to the invention, the first pumping means 8 comprise both a centrifugal pump and a peristaltic pump, the supply line 2 being connectable by the medical staff to one or the other pump depending on the type of therapy one wishes to follow. More in particular, in the case in which the therapy to be followed is of long duration, the supply line 2 is connected to the centrifugal pump, while in the case of a shorter therapy the supply line 2 is connected to the peristaltic pump.

The connection of the supply line 2 to the centrifugal pump or to the peristaltic pump is of the selective type, or the supply line 2 is connected from time to time only to the centrifugal pump, or only to the peristaltic pump depending on the type of treatment to be performed.

Advantageously, the supply line 2 comprises a main section, identified in the figures with the reference numeral 2a, connectable on one side to the patient and connected on the opposite side to the input of the oxygenation device 3, and a by-pass section, identified in the figures with the reference numeral 2b, arranged parallel to the main section 2a and along which the filtering device 4 is arranged.

The by-pass section 2b therefore has a delivery branch connected on one side to the main section 2a and on the opposite side to the input of the filtering device 4, and a return branch connected on one side to the output of the filtering device 4 and on the opposite side to the main section 2a. The delivery branch and the return branch are identified in the figures with reference numerals 6 and 7, respectively.

More particularly, the first pumping means 8 are arranged along the main section 2a and are adapted to convey the blood to be treated towards the oxygenation device 3.

The first pumping means 8 are arranged upstream of the by-pass section 2b, i.e. upstream of the delivery branch 6, with respect to the direction of advancement of the blood towards the oxygenation device 3.

Conveniently, the apparatus 1 also comprises second pumping means 9 arranged along the by-pass section 2b and adapted to convey the blood towards the filtering device 4.

Preferably, removable connection means 10 are arranged along the delivery branch 6 and along the return branch 7, which are adapted to allow the separation of a first portion 11 of each of the branches 6 and 7 from a relevant second portion 12, where the second portions 12 are connectable to each other by means of the connection means themselves following the separation from the relevant first portions 11.

By joining the second portions 12 together, a section is therefore defined that extends parallel to the main section 2a and which is devoid of blood treatment devices.

Conveniently, the second pumping means 9 are arranged along the first portion 11 of the delivery branch 6, upstream of the filtering device 4 with respect to the direction of blood flow.

Along the by-pass section 2b, and more particularly along the relevant second portions 12, closing means 13 are arranged, of the type of clamps or the like, operable to block the relevant blood passage channels and consequently isolate the filtering device 4. The closing means 13 are therefore arranged along the delivery branch 6 and along the return branch 7, upstream and downstream respectively of the relevant connection means 10.

Following the mutual linkage of the connection means 10, the closing means 13 are operated again to allow the passage of blood through the relevant second portions 12.

The apparatus 1 also comprises a discharge line 14 of the waste substances filtered by the filtering device 4, also called ultrafiltrate, which is connected on one side to the filtering device itself and on the other side to a collection bag 15.

Preferably an infusion line 16 is also provided of at least one balancing or restoring substance connected to the by-pass section 2b.

More particularly, the infusion line 16 is connected on one side to a bag 17 containing the balancing substance to infuse and on the opposite side to the by-pass section 2b, at the first portion 11 of the return branch 7, downstream of the filtering device 4.

Conveniently, third pumping means 18 are arranged along the infusion line 16 adapted to convey the balancing substance from the bag 17 towards the by-pass section 2b.

The operation of the present invention is as follows.

In normal operating conditions, i.e. in the case in which the oxygenation device 3 and the filtering device 4 are both functioning, the blood taken from the patient is sent through the first pumping means 8 along the main section 2a towards the oxygenation device 3.

More in detail, one part of the blood that runs through the main section 2a reaches the oxygenation device 3 and one part is deflected, through the second pumping means 9, along the by-pass section 2b. The part of blood that runs through the delivery branch 6 of the by-pass section 2b is therefore sent by the second pumping means 9 towards the filtering device 4, later on going back inside the main section 2a by means of the return branch 7.

When it is necessary to replace the filtering device 4, in order to maintain the oxygenation device 3 functioning, the by-pass section 2b is blocked by means of the closing means 13.

Subsequently the first portions 11 together with the filtering device 4 are removed from the connection means 10 and the second portions 12 are connected to one another. The second portions 12 connected in this manner then define a section parallel to the main section 2a and devoid of blood treatment devices. Once the passage channel defined by the second portions 12 is reopened, again through the closing means 13, the blood taken from the patient is then entirely sent to the oxygenation device 3 by means of the first pumping means 8.

When the operator wishes to restore the complete functionality of the apparatus 1, by inserting again the filtering device 4, he/she should perform the aforementioned operations but in the reverse sequence. More in detail, the second portions 12 are blocked by means of the closing means 13, so as to prevent the passage of blood through the by-pass section 2b, and the second portions are separated from each other by means of the connection means 10.

Subsequently the first portions 11, along which a new filtering device 4 and the second pumping means 9 are positioned, are connected again to the relevant second portions 12 by means of the connection means 10.

It has in practice been ascertained that the described invention achieves the intended objects and in particular the fact is underlined that the use of a centrifugal pump to convey the blood through the oxygenation device and/or the filtering device allows performing treatments also of long duration.

More in particular, the presence of both a centrifugal pump and of a peristaltic pump allows the medical staff to be able to choose the type of therapy to be followed in the specific case.

A further advantage of the apparatus to which the present invention relates consists in the fact that the presence of the connection means arranged along the by-pass section allows separating the latter from the main section, in order to allow the replacement of the filtering device, at the same time maintaining the oxygenation device functioning.

## Claims

1. Apparatus (1) for the decapneization of blood, comprising:
- a supply line (2) of the blood to be treated taken from a patient and at least a return line (5) adapted to transport the treated blood to the patient;
- at least an oxygenation device (3) and at least a filtering device (4) of the blood positioned between said supply line (2) and said return line (5);
- first pumping means (8) adapted to convey the blood along said supply line (2) towards at least one of said oxygenation device (3) and said filtering device (4);
**characterized by** the fact that said first pumping means (8) comprise at least a centrifugal pump and at least a peristaltic pump and by the fact that said supply line (2) is selectively connectable to one of said centrifugal pump and said peristaltic pump.

2. Apparatus (1) according to claim 1, **characterized by** the fact that said supply line (2) comprises at least one main section (2a), connectable on one side to the patient and connected on the opposite side to said oxygenation device (3), and at least a by-pass section (2b), arranged parallel to said main section (2a) and along which said filtering device (4) is positioned, and by the fact that said first pumping means (8) are arranged along said main section (2a) upstream of said by-pass section.

3. Apparatus (1) according to one or more of the preceding claims, **characterized by** the fact that it comprises second pumping means (9) arranged along said by-pass section (2b) and adapted to convey the blood to be treated towards said filtering device (4).

4. Apparatus (1) according to one or more of the preceding claims, **characterized by** the fact that said by-pass section (2b) comprises a delivery branch (6), connected on one side to said main section (2a) and on the other side to the input of said filtering device (4), and a return branch (7), connected on one side to the output of the filtering device (4) and on the other side to said main section (2a), and by the fact that it comprises removable connection means (10), arranged along said branches (6, 7) and adapted to allow the separation of a first portion (11) of the branches themselves which is connected to said filtering device (4), from a respective second portion (12) connected to said main section (2a), said second portions (12) being connectable to each other by means of the connection means themselves following the separation from their respective first portions (11).

5. Apparatus (1) according to claim 4, **characterized by** the fact that said second pumping means (9) are arranged downstream of said connection means (10) along the first portion (11) of said delivery branch (6) and upstream of the filtering device (4).

6. Apparatus (1) according to claim 4 or 5, **characterized by** the fact that it comprises closing means (13) of said by-pass section (2b) arranged along said second portions (12).

7. Apparatus (1) according to one or more of the preceding claims, **characterized by** the fact that it comprises at least a discharge line (14) of the waste substances filtered by said filtering device (4), connected on one side to the filtering device itself and connectable on the other side to a collection bag (15).

8. Apparatus (1) according to one or more of the preceding claims, **characterized by** the fact that it comprises at least one infusion line (16) of at least one balancing substance connected to said by-pass section (2b).

9. Apparatus (1) according to claim 8, **characterized by** the fact that said infusion line (16) is connected to said by-pass section (2b) at the first portion (11) of said return branch (7), downstream of said filtering device (4).

10. Apparatus (1) according to claim 8 or 9, **characterized by** the fact that it comprises third pumping means (18) arranged along said infusion line (16).

## Patentansprüche

1. Vorrichtung (1) zur Decapneisierung von Blut, umfassend:
- eine Zuführungsleitung (2) für das zu behandelnde Blut, das einem Patienten entnommen wurde, und mindestens eine Rücklaufleitung (5), die eingerichtet ist, das behandelte Blut zu dem Patienten zu transportieren;
- mindestens eine Oxygenierungsvorrichtung (3) und mindestens eine Filtervorrichtung (4) des Blutes, die zwischen der Zuführungsleitung (2) und der Rücklaufleitung (5) angeordnet ist;
- erste Pumpmittel (8), die eingerichtet sind, um das Blut entlang der Zuführungsleitung (2) in Richtung von mindestens einem von der Oxygenierungsvorrichtung (3) und der Filtervorrichtung (4) zu transportieren;
**dadurch gekennzeichnet, dass** die ersten Pumpmittel (8) mindestens eine Zentrifugalpumpe und mindestens eine peristaltische Pumpe umfassen und dass die Zuführungsleitung (2) wahlweise mit einer von der Zentrifugalpumpe und der peristaltischen Pumpe verbindbar ist.

2. Vorrichtung (1) nach Anspruch 1, **dadurch gekennzeichnet, dass** die Zuführungsleitung (2) mindestens einen Hauptabschnitt (2a), der auf einer Seite mit dem Patienten verbindbar und auf der gegenüberliegenden Seite mit der Oxygenierungsvorrichtung (3) verbunden ist, und mindestens einen Umgehungsabschnitt (2b) umfasst, der parallel zu dem Hauptabschnitt (2a) angeordnet ist und entlang dem die Filtervorrichtung (4) angeordnet ist, und dadurch, dass die ersten Pumpmittel (8) entlang des Hauptabschnitts (2a) stromaufwärts des Umgehungsabschnitts angeordnet sind.

3. Vorrichtung (1) nach einem oder mehreren der vorangehenden Ansprüche, **dadurch gekennzeichnet, dass** sie zweite Pumpmittel (9) umfasst, die entlang des Umgehungsabschnitts (2b) angeordnet und eingerichtet sind, um das zu behandelnde Blut in Richtung der Filtervorrichtung (4) zu transportieren.

4. Vorrichtung (1) nach einem oder mehreren der vorangehenden Ansprüche, **dadurch gekennzeichnet, dass** der Umgehungsabschnitt (2b) einen Abgabezweig (6), der auf der einen Seite mit dem Hauptabschnitt (2a) und auf der anderen Seite mit dem Eingang der Filtervorrichtung (4) verbunden ist, und einen Rücklaufzweig (7) umfasst, der auf der einen Seite mit dem Ausgang der Filtervorrichtung (4) und auf der anderen Seite mit dem Hauptabschnitt (2a) verbunden ist, und dadurch, dass er abnehmbare Verbindungsmittel (10) umfasst, die entlang der Zweige (6, 7) angeordnet sind und dazu eingerichtet sind, die Trennung eines ersten Abschnitts (11) der Zweige selbst, der mit der Filtervorrichtung (4) verbunden ist, von einem entsprechenden zweiten Abschnitt (12), der mit dem Hauptabschnitt (2a) verbunden ist, zu ermöglichen, wobei die zweiten Abschnitte (12) mittels der Verbindungsmittel selbst im Anschluss an die Trennung von ihren entsprechenden ersten Abschnitten (11) miteinander verbindbar sind.

5. Vorrichtung (1) nach Anspruch 4, **dadurch gekennzeichnet, dass** die zweiten Pumpmittel (9) stromabwärts der Verbindungsmittel (10) entlang des ersten Abschnitts (11) des Abgabezweiges (6) und stromaufwärts der Filtervorrichtung (4) angeordnet sind.

6. Vorrichtung (1) nach Anspruch 4 oder 5, **dadurch gekennzeichnet, dass** sie Verschlussmittel (13) des Umgehungsabschnittes (2b) umfasst, die entlang der zweiten Abschnitte (12) angeordnet sind.

7. Vorrichtung (1) nach einem oder mehreren der vorangehenden Ansprüche, **dadurch gekennzeichnet, dass** sie mindestens eine Ablaufleitung (14) der von der Filtervorrichtung (4) gefilterten Abfallsubstanzen umfasst, die auf einer Seite mit der Filtervorrichtung selbst verbunden und auf der anderen Seite mit einem Sammelbeutel (15) verbindbar ist.

8. Vorrichtung (1) nach einem oder mehreren der vorangehenden Ansprüche, **dadurch gekennzeichnet, dass** sie mindestens eine Infusionsleitung (16) von mindestens einer Ausgleichssubstanz umfasst, die mit dem Umgehungsabschnitt (2b) verbunden ist.

9. Vorrichtung (1) nach Anspruch 8, **dadurch gekennzeichnet, dass** die Infusionsleitung (16) mit dem Umgehungsabschnitt (2b) an dem ersten Abschnitt (11) des Rücklaufzweiges (7), stromabwärts der Filtervorrichtung (4), verbunden ist.

10. Vorrichtung (1) nach Anspruch 8 oder 9, **dadurch gekennzeichnet, dass** sie dritte Pumpmittel (18) umfasst, die entlang der Infusionsleitung (16) angeordnet sind.

## Revendications

1. Appareil (1) d'élimination du gaz carbonique dans le sang, comprenant :
- une ligne d'alimentation (2) du sang à traiter prélevé d'un patient et au moins une ligne de retour (5) adaptée à transporter le sang traité au patient ;
- au moins un dispositif d'oxygénation (3) et au moins un dispositif de filtration (4) du sang positionné entre ladite ligne d'alimentation (2) et ladite ligne de retour (5) ;
- un premier moyen de pompage (8) adapté à acheminer le sang le long de ladite ligne d'alimentation (2) en direction d'au moins l'un dudit dispositif d'oxygénation (3) et dudit dispositif de filtration (4) ;
**caractérisé par le fait que** ledit premier moyen de pompage (8) comprend au moins une pompe centrifuge et au moins une pompe péristaltique et **par le fait que** ladite ligne d'alimentation (2) est sélectivement raccordable à l'une de ladite pompe centrifuge et de ladite pompe péristaltique.

2. Appareil (1) selon la revendication 1, **caractérisé par le fait que** ladite ligne d'alimentation (2) comprend au moins une section principale (2a), raccordable sur un côté au patient et raccordée sur le côté opposé audit dispositif d'oxygénation (3), et au moins une section de dérivation (2b), agencée parallèle à ladite section principale (2a) et le long de laquelle ledit dispositif de filtration (4) est positionné, et **par le fait que** ledit premier moyen de pompage (8) est agencé le long de ladite section principale (2a) en amont de ladite section de dérivation.

3. Appareil (1) selon une ou plusieurs des revendications précédentes, **caractérisé par le fait qu'**il comprend un second moyen de pompage (9) agencé le long de ladite section de dérivation (2b) et adapté à acheminer le sang à traiter en direction dudit dispositif de filtration (4).

4. Appareil (1) selon une ou plusieurs des revendications précédentes, **caractérisé par le fait que** ladite section de dérivation (2b) comprend une branche de distribution (6), raccordée sur un côté à ladite section principale (2a) et sur l'autre côté à l'entrée dudit dispositif de filtration (4), et une branche retour (7), raccordée sur un côté à la sortie du dispositif de filtration (4) et sur l'autre côté à l'entrée de ladite section principale (2a), et **par le fait qu'**il comprend un moyen de raccordement amovible (10), agencé le long desdites branches (6, 7) et adapté à permettre la séparation d'une première partie (11) des branches elles-mêmes qui est raccordée au dit dispositif de filtration (4), d'une seconde partie respective (12) raccordée à ladite section principale (2a), lesdites secondes parties (12) pouvant être raccordées les unes aux autres via le moyen de raccordement lui-même après la séparation de leurs premières parties respectives (11).

5. Appareil (1) selon la revendication 4, **caractérisé par le fait que** ledit second moyen de pompage (9) est agencé en aval dudit moyen de raccordement (10) le long de la première partie (11) de ladite branche de distribution (6) et en amont dudit dispositif de filtration (4).

6. Appareil (1) selon la revendication 4 ou 5, **caractérisé par le fait qu'**il comprend un moyen de fermeture (13) de ladite section de dérivation (2b) agencé le long desdites secondes parties (12).

7. Appareil (1) selon une ou plusieurs des revendications précédentes, **caractérisé par le fait qu'**il comprend au moins une ligne de décharge (14) des déchets filtrés par ledit dispositif de filtration (4), raccordée sur un côté au dispositif de filtration lui-même et raccordable sur l'autre côté à une poche de collecte (15).

8. Appareil (1) selon une ou plusieurs des revendications précédentes, **caractérisé par le fait qu'**il comprend au moins une ligne de perfusion (16) d'au moins une substance d'équilibrage raccordée à ladite section de dérivation (2b).

9. Appareil (1) selon la revendication 8, **caractérisé par le fait que** ladite ligne de perfusion (16) est raccordée à ladite section de dérivation (2b) au niveau de la première partie (11) de ladite branche de retour (7), en aval dudit dispositif de filtration (4).

10. Appareil (1) selon la revendication 8 ou 9, **caractérisé par le fait qu'**il comprend un troisième moyen de pompage (18) agencé le long de ladite ligne de perfusion (16).
